# FASCICULE DE BREVET EUROPEEN

(11) **EP 2 441 344 B1**
(45) Date de publication et mention de la délivrance du brevet: **06.12.2017**
(21) Numéro de dépôt: 11184724.0
(22) Date de dépôt: 11.10.2011
(51) Int. Cl.: A45D 34/02, B05B 11/00, A61M 11/00, B65B 3/14

(54) **Procédé et flacon de distribution d'un produit fluide**
Verfahren und Spendefläschchen für ein Flüssigprodukt
Method and bottle for dispensing a fluid product

(30) Priorité: 18.10.2010 FR 1004092
(43) Date de publication de la demande: 18.04.2012
(73) Titulaire: Albéa le Tréport, 76470 Le Tréport (FR)
(72) Inventeur: Dumont, Pierre, 80570 Dargnies (FR); Maduit, Emmanuel, 76260 Eu (FR); Octau, Jean-Luc, 76630 Intraville (FR)
(74) Mandataire: Gevers & Orès

(56) Documents cités:
- EP-B1- 0 626 210
- CH-A- 258 147
- DE-A1- 2 651 869
- DE-B- 1 087 513
- FR-A1- 2 854 131

## Description

L'invention concerne un procédé de distribution d'un produit fluide au moyen d'un flacon de distribution ainsi qu'un flacon de distribution d'un tel produit. En particulier, le produit peut être liquide, par exemple cosmétique de soin, de maquillage ou de parfumage, ou encore pharmaceutique.

Le flacon de distribution comprend un corps rigide dans lequel un réservoir de conditionnement du produit est formé, ainsi qu'un dispositif de prélèvement du produit conditionné qui est monté de façon étanche sur ledit corps. En particulier, le dispositif de prélèvement peut comprendre une pompe à actionnement manuel qui est alimentée en produit conditionné, ladite pompe étant agencée pour distribuer le produit sous pression, par exemple sous la forme d'un aérosol.

Dans un exemple d'application, les flacons selon l'invention permettent la distribution d'échantillons de produit, notamment pour un volume de produit conditionné dans le réservoir qui est compris entre 1 et 10 ml. En particulier, les échantillons ainsi distribués peuvent permettre à un client de tester le produit, les flacons étant alors qualifiés de flacons testeurs d'échantillons. En variante, les flacons peuvent être dits « de sac » en ce qu'ils permettent de transporter facilement un volume réduit de produit, par opposition à des flacons de contenance supérieure qui sont en général lourds et encombrants car cossus.

Dans ces applications, par exemple pour des raisons logistiques, de praticité ou encore environnementales de recyclage, il peut être souhaitable de pouvoir recharger le réservoir en produit à partir d'une source dudit produit. En effet, il est peu pratique pour un utilisateur d'effectuer le remplissage du réservoir à l'aide d'un petit entonnoir et peu écologique de jeter un flacon vide pour le remplacer par un plein constituant recharge.

Des flacons de distribution sont déjà proposés à la vente, dans lesquels le corps est équipé d'une soupape de remplissage du réservoir qui est agencée pour permettre la mise en communication d'une source de produit avec ledit réservoir. En particulier, la soupape peut s'ouvrir par appui sur le gicleur de la pompe d'un flacon source qu'il convient d'actionner à plusieurs reprises pour réaliser le remplissage, ce qui est un geste peu intuitif pour l'utilisateur.

On connaît également, notamment du document FR-2 854 131, un procédé de remplissage d'un réservoir qui prévoit le vidage préalable dudit réservoir au moyen d'une pompe de distribution sans reprise d'air de sorte à créer une dépression dans ledit réservoir, puis la mise en communication d'une source de produit avec ladite pompe afin de réaliser le remplissage par aspiration au travers de ladite pompe. Toutefois, cette réalisation nécessite l'utilisation d'une pompe spécifique qui permet à la fois le remplissage et la distribution.

En outre, cette réalisation ne permet pas le remplissage initial du flacon de distribution de façon satisfaisante, et ce que ce soit par les utilisateurs et/ou par les distributeurs sur le lieu de vente. En effet, notamment lors de l'assemblage du flacon dans l'usine de conditionnement, un remplissage initial du réservoir en produit doit être réalisé préalablement au rechargement par aspiration. Ainsi, les distributeurs de ces flacons doivent disposer d'un flacon spécifique pour chacun des produits qu'il propose à la vente.

En particulier, pour la distribution d'échantillons de produit, un flacon testeur d'échantillon doit être prévu pour chacun des produits puisque que le remplissage dudit flacon ne peut pas être réalisé simplement au moment de sa remise au client. Aussi, les distributeurs de produits disposent d'un nombre considérable de flacons testeurs d'échantillons qui se ressemblent tous et parmi lesquels ils doivent trouver celui qu'ils recherchent pour le remettre à un client.

Par ailleurs, certaines marques peuvent souhaiter que les flacons de leur produit ne soient pas remplissables ultérieurement par les utilisateurs, tout en bénéficiant d'une simplification du remplissage initial dudit flacon. En particulier, il peut être requis que les flacons testeurs d'échantillons ne soient remplissables qu'une seule fois.

On connaît du document DE-2 651 869 un procédé selon le préambule de la revendication 1 et un flacon selon le préambule de la revendication 13.

L'invention vise à perfectionner l'art antérieur en proposant notamment un procédé et un flacon de distribution dont le remplissage initial en produit peut être réalisé de façon particulièrement simple et modulable, notamment chez les distributeurs, et ce sans obligation de pouvoir remplir ultérieurement ledit flacon ni d'utiliser un dispositif de prélèvement spécifique.

A cet effet, et selon un premier aspect, l'invention propose un procédé de distribution selon la revendication 1.

Selon un deuxième aspect, l'invention propose un flacon de distribution d'un produit fluide selon la revendication 13.

D'autres objets et avantages de l'invention apparaîtront dans la description qui suit, faite en référence aux figures annexées, dans lesquelles :
- la figure 1 est une vue en coupe longitudinale d'un flacon de distribution selon un mode de réalisation de l'invention ;
- la figure 2 est une vue en coupe longitudinale d'un flacon de distribution selon un mode de réalisation de l'invention, la figure 2a étant une vue agrandie de la zone A de la figure 2 ;
- la figure 3 est une vue en coupe longitudinale d'un flacon de distribution selon un mode de réalisation de l'invention, la figure 3a étant une vue agrandie de la zone A de la figure 3 ;
- la figure 4 est une vue en coupe longitudinale d'un flacon de distribution selon un mode de réalisation de l'invention dans laquelle le dispositif de prélèvement est en position de stockage, la figure 4a étant une vue agrandie de la zone A de la figure 4 ;
- les figures 5 représentent en coupe longitudinale l'extrémité inférieure d'un flacon de distribution qui est équipée d'une soupape de remplissage, ladite soupape étant respectivement en état stable de fermeture étanche (figure 5a) et en état contraint de mise en communication étanche avec un tube de prélèvement d'une source de produit (figure 5b) ;
- les figures 6 sont des vues en coupe longitudinale d'un flacon de distribution selon un mode de réalisation de l'invention, la soupape de remplissage étant montée sur le gicleur de la pompe d'un flacon source de produit, respectivement avant remplissage en état stable de fermeture étanche (figure 6a) et en cours de remplissage en état contraint de mise en communication (figure 6b).

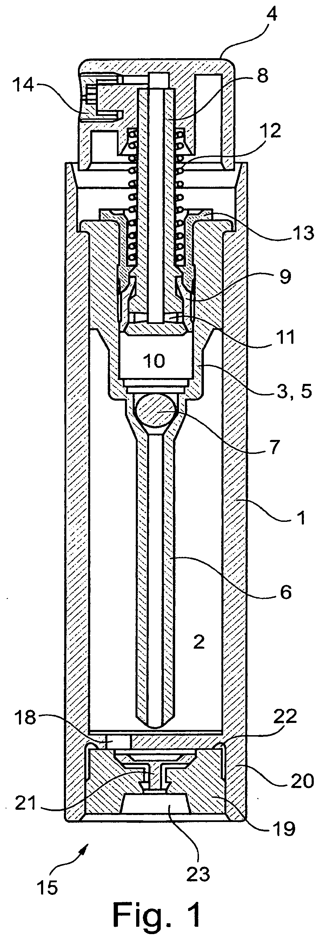

Dans la description, les termes de positionnement dans l'espace sont pris en référence à la position du flacon représenté sur la figure 1.

En relation avec les figures, on décrit ci-dessous un flacon destiné à contenir un produit fluide en vue de sa distribution, ainsi qu'un procédé de distribution utilisant un tel flacon. Dans des exemples particuliers, le produit peut être liquide, notamment un produit cosmétique de soin, de maquillage ou de parfumage, ou un produit pharmaceutique.

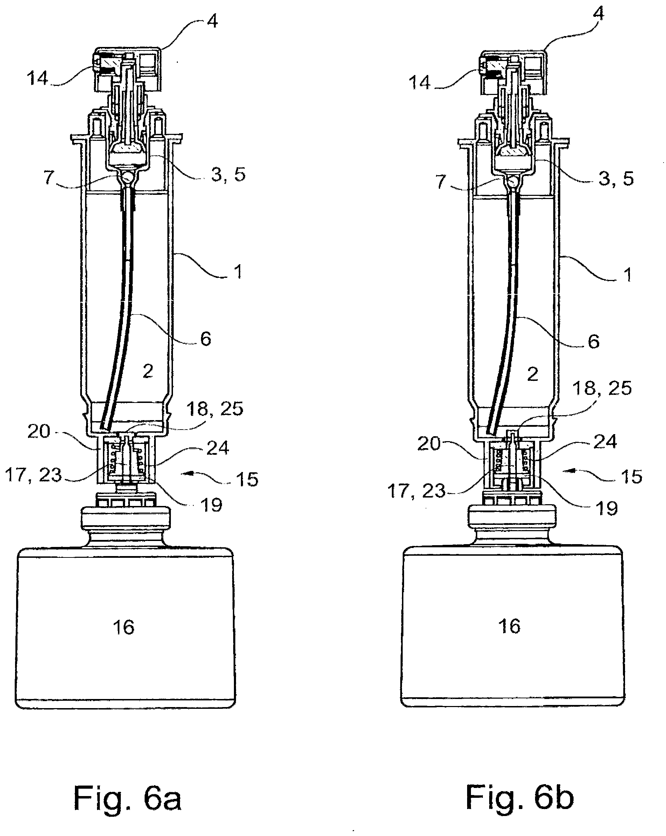

Le flacon comprend un corps rigide 1 dans lequel un réservoir 2 de conditionnement du produit est formé. En particulier, le corps 1 présente une rigidité suffisante pour que le volume du réservoir 2 demeure sensiblement constant. Selon une application particulière, le réservoir 2 peut avoir une contenance comprise entre 1 et 10 ml de sorte à permettre la distribution d'échantillons de produit. En relation avec les figures 6, le corps 1 est destiné à être inséré dans un habillage (non représenté).

Le corps 1 peut être monobloc, par exemple réalisé par injection-soufflage ou extrusion-soufflage, ou en plusieurs parties injectées puis assemblées, par exemple par soudure ultra-sons, en matière plastique rigide, en métal, par exemple en aluminium, ou en verre.

Le flacon comprend également un dispositif de prélèvement 3 du produit conditionné qui est monté de façon étanche sur le corps 1, notamment dans l'ouverture supérieure dudit corps. Dans les modes de réalisation représentés, le dispositif de prélèvement comprend une pompe 3 de distribution actionnée manuellement au moyen d'un bouton poussoir 4 qui est alimenté avec le produit sous pression en vue de sa distribution.

La pompe 3 comprend un corps 5 équipé de moyens d'alimentation en produit conditionné. Sur les figures, les moyens d'alimentation comprennent un tube plongeur 6 disposé dans le réservoir 2, ledit tube étant équipé d'un clapet 7 d'entrée du produit dans la pompe 3. Le bouton poussoir 4 est monté sur le gicleur 8 de la pompe 3 qui comprend un piston 9 monté autour dudit gicleur pour délimiter dans le corps 1 une chambre de dosage 10. Le piston 9 permet l'ouverture - respectivement la fermeture - des orifices 11 d'alimentation du gicleur 8 sur une course de distribution - respectivement d'aspiration - dudit gicleur.

Le bouton poussoir 4 comprend une zone supérieure permettant à l'utilisateur d'exercer un appui digital sur ledit bouton poussoir afin de pouvoir le déplacer axialement sur sa course d'actionnement de la pompe 3, le retour du bouton poussoir 4 sur sa course d'aspiration étant classiquement réalisé par un ressort 12. Dans le mode de réalisation représenté, l'intérieur du corps 5 de pompe 3 est équipé d'un extenseur 13 sur lequel l'extrémité inférieure du ressort 12 est en appui.

Le bouton poussoir 4 est équipé d'une buse de pulvérisation 14 qui est agencée pour distribuer radialement un aérosol du produit. Toutefois, l'invention n'est pas limitée à un mode particulier de distribution du produit. En particulier, notamment pour un embout nasal de pulvérisation, le bouton poussoir 4 peut permettre une distribution axiale du produit et un autre type de dispositif de prélèvement peut être envisagé.

Le corps 1 du flacon est équipé d'une soupape 15 de remplissage du réservoir 2 qui est agencée pour permettre la mise en communication d'une source de produit 16 avec ledit réservoir. La source de produit 16 peut comprendre un réservoir source sur lequel est disposé un tube de prélèvement 17, le remplissage en produit du réservoir 2 étant réalisé par montage dudit tube en appui étanche sur la soupape 15 qui est agencée pour s'ouvrir de façon réversible.

En particulier, on peut utiliser en tant que source de produit 16 un flacon nourrice de contenance supérieure (figures 6), ledit flacon étant équipé d'une pompe dont le bouton poussoir est retiré pour permettre la disposition du gicleur en appui étanche sur la soupape 15. En effet, outre l'ouverture de la soupape 15, l'appui étanche provoque l'ouverture de la pompe afin de permettre le passage du produit de remplissage au travers d'elle.

Selon une autre réalisation, le réservoir source est formé à l'intérieur d'une poche souple qui peut être remplie de produit sans air ni gaz pour la bonne conservation dudit produit. Le transfert du produit dans le réservoir 2 est alors possible dans toutes les positions et la poche souple ne peut pas être détournée de son rôle de source puisque sans gaz propulseur ni pression interne, ni bouton poussoir pour actionner une éventuelle pompe associée au tube de prélèvement 17.

Dans les modes de réalisation représentés, la soupape 15 de remplissage est disposée sur l'extrémité inférieure du corps 1, de sorte notamment à remplir le réservoir 2 par le fond du flacon, ce qui correspond à un geste intuitif.

Pour ce faire, l'extrémité inférieure du corps 1 présente un orifice 18 de communication avec le réservoir 2 et la soupape 15 présente un siège 19 qui est mobile et/ou déformable entre un état stable de fermeture étanche de l'orifice 18 du réservoir 2 et un état contraint d'ouverture dudit orifice pour la mise en communication étanche de la source de produit 16 avec ledit réservoir.

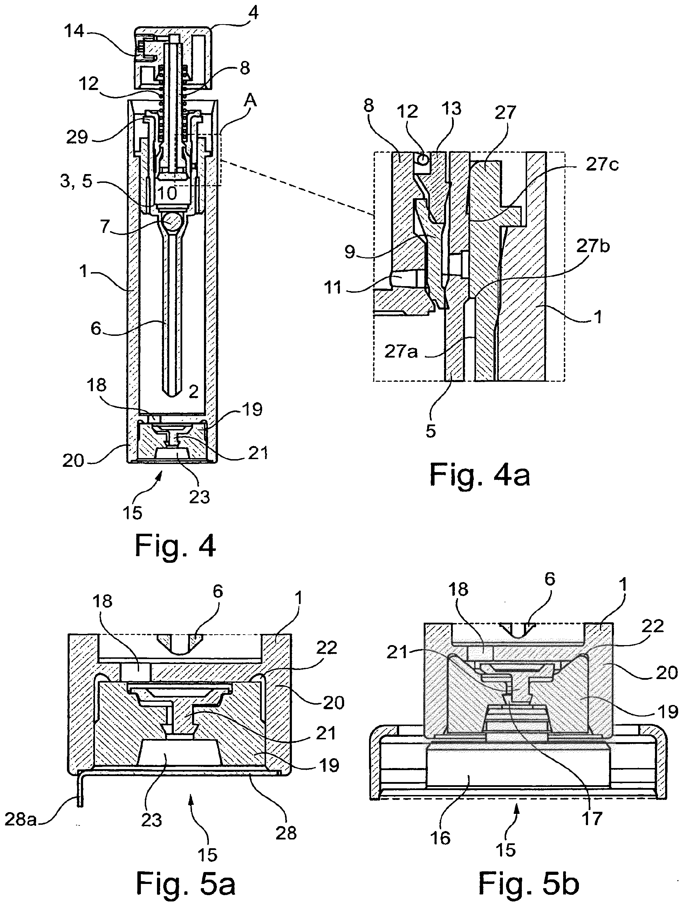

En relation avec les figures 1 à 5, le corps 1 présente un enjoliveur inférieur 20 dans lequel un clapet rigide 21 est formé, ledit enjoliveur présentant un fond dans lequel l'orifice 18 est formé, ledit fond présentant une gorge périphérique 22. La soupape 15 comprend également un siège 19 déformable, par exemple réalisé en matériau élastomérique, qui présente un logement inférieur 23 d'entrée du tube de prélèvement 17, ledit siège en état stable de fermeture étant en appui étanche sur le clapet 21 (figure 5a). L'appui du tube de prélèvement 17 dans le siège 19 induit le fluage réversible dudit siège dans la gorge 22 (figure 5b) de sorte à desserrer l'appui étanche et donc rompre l'étanchéité entre ledit siège et le clapet 21 afin de mettre le logement 23 en communication avec l'orifice 18.

En relation avec les figures 6, la soupape 15 comprend un siège 19 mobile pourvu d'un logement inférieur 23 d'entrée du tube de prélèvement 17, ledit siège étant monté en translation réversible dans l'enjoliveur 20 au moyen d'un ressort de rappel 24. Le siège 19 présente un embout 25 qui est monté dans l'orifice 18, ledit embout étant pourvu de passages qui sont respectivement fermés en position stable et ouverts en position enfoncée afin de mettre le logement 23 en communication avec ledit orifice.

Le procédé de distribution prévoit, préalablement au remplissage initial du réservoir 2 en produit, de mettre ledit réservoir vide de produit en communication avec un dispositif d'aspiration d'air et d'activer ledit dispositif afin de créer une dépression à l'intérieur dudit réservoir.

Selon une réalisation, le dispositif d'aspiration d'air comprend une cloche à vide dans laquelle le flacon de distribution est disposé, le montage étanche du dispositif de prélèvement 3 sur le corps 1 étant réalisé après activation de ladite cloche. Ainsi, la dépression est formée dans le réservoir 2 puis le dispositif de prélèvement 3 est monté de façon étanche pour maintenir ladite dépression.

Selon une autre réalisation, le dispositif d'aspiration d'air, par exemple une pompe à vide, est mis en communication avec le dispositif de prélèvement 3 après son montage étanche sur le corps 1, l'aspiration de l'air du réservoir 2 étant réalisée au travers dudit dispositif de prélèvement. En variante, l'aspiration d'air pourrait être réalisée au travers de la soupape 15 en prévoyant de la mettre en communication avec le dispositif d'aspiration d'air après montage étanche du dispositif de prélèvement 3 sur le corps 1.

Le procédé de distribution prévoit de réaliser ultérieurement le remplissage initial du réservoir 2 par mise en communication étanche d'une source de produit 16 avec ledit réservoir par l'intermédiaire de la soupape 15 de sorte que la dépression induise le remplissage dudit réservoir par aspiration du produit contenu dans ladite source. Ensuite, le client peut actionner le dispositif de prélèvement 3 pour distribuer le produit conditionné.

En effet, après disposition du tube de prélèvement 17 dans le logement 23, un seul appui induit l'ouverture de la soupape 15, ainsi qu'éventuellement l'ouverture de la pompe du flacon source 16, de sorte à former un chemin de transfert de produit entre la source 16 et le réservoir 2. La compensation de la dépression permet alors le remplissage. Ensuite, lorsque le réservoir 2 est rempli, la dépression d'aspiration devient nulle et la soupape 15 est alors refermée lors du retrait du tube de prélèvement 17 et le produit contenu dans le réservoir 2 peut être distribué ultérieurement par l'intermédiaire du dispositif de prélèvement 3.

Le flacon de distribution qui est livré aux distributeurs est donc vide de produit et présente une dépression d'air, ladite dépression permettant de réaliser ultérieurement le remplissage initial, notamment au moment de la remise du flacon au client en fonction du produit qu'il souhaite acheter et/ou tester. Le procédé permet donc un remplissage initial particulièrement modulable, qui permet en particulier une gestion simplifiée des flacons par les distributeurs, notamment des flacons testeurs d'échantillons.

La modularité du procédé de distribution peut également être améliorée en prévoyant l'association d'une étiquette sur le corps 1 au moment du remplissage initial du réservoir 2 en produit, notamment en fonction dudit produit. L'étiquette peut présenter une partie détachable comprenant une offre commerciale propre au distributeur afin d'encourager le client à revenir acheter le produit échantillonné.

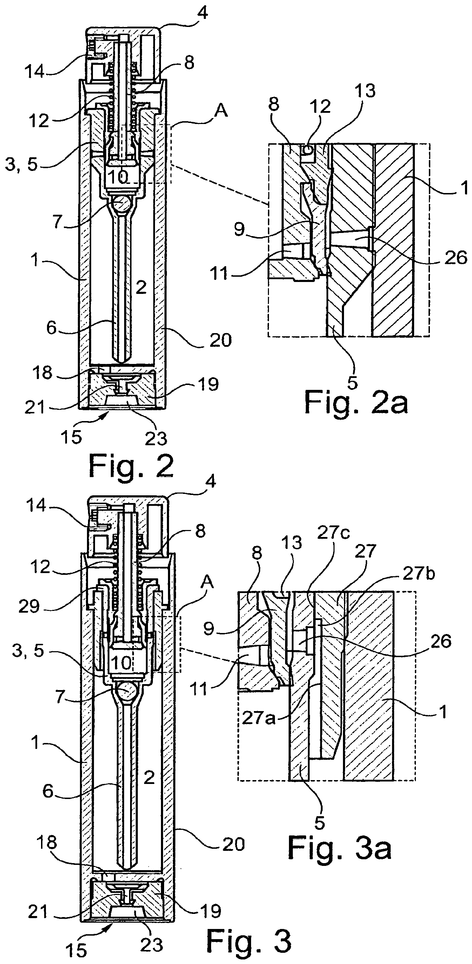

En relation avec les figures 1 et 2, le dispositif de prélèvement 3 est du type sans reprise d'air (airless en anglais) dans le réservoir 2 en compensation du volume de produit distribué. Pour ce faire, le corps 5 de la pompe 3 de la figure 1 est dépourvu de trou d'évent.

Toutefois, les pompes 3 avec trou d'évent 26 étant les plus courantes, il peut être avantageux de créer une dépression dans le réservoir 2 même avec ce type de pompe. Pour ce faire, la figure 2 présente un corps 5 pourvu d'un trou d'évent 26 qui est occulté de façon étanche par son montage dans le corps rigide 1 (figure 2a). En particulier, l'étanchéité entre le corps 5 et le corps rigide 1 est alors faite au moins en dessous du trou d'évent 26 de sorte à empêcher le passage d'air depuis la pompe 3 dans le réservoir 2 par l'intermédiaire dudit trou. Sur la figure 2, l'étanchéité est également réalisée au dessus du trou d'évent 26, ce qui ne nuit pas au fonctionnement sans reprise d'air et est un peu plus simple à réaliser.

Le produit prélevé sans reprise d'air dans le réservoir 2 permet de créer dans ledit réservoir une dépression qui augmente au fur et à mesure de la distribution. En particulier, pour assurer un vidage total du réservoir 2, le ciel d'air au dessus du produit lors du remplissage initial doit être tel que la dépression atteinte en fin de vidage soit au maximum égale à la dépression réalisable par la pompe 3.

Dans cette réalisation, le procédé de distribution prévoit alors la possibilité de remplissage ultérieur du réservoir 2 par mise en communication étanche de la source de produit 16 avec ledit réservoir par l'intermédiaire de la soupape 15 de sorte que la dépression induise le remplissage dudit réservoir par aspiration du produit contenu dans ladite source.

En relation avec les figures 3 et 4, le produit est prélevé avec reprise d'air dans le réservoir 2 de sorte à empêcher le remplissage ultérieur dudit réservoir par aspiration. Pour ce faire, la pompe 3 comprend un trou d'évent 26 qui est agencé pour permettre de compenser le volume de produit prélevé dans le réservoir 2 par de l'air.

En relation avec la figure 3, la pompe 3 est montée dans le corps 1 par l'intermédiaire d'un manchon 27 dans lequel le corps 5 est emmanché de façon étanche. Le trou d'évent 26 est formé dans le corps 2 en regard radial d'un diamètre augmenté 27a qui est formé sur le manchon 27 de sorte à laisser libre ledit trou pour permettre la reprise d'air (figure 3a) dans le réservoir 2.

Selon une réalisation, le procédé de distribution peut prévoir, préalablement au remplissage initial du réservoir 2 en produit, le montage du dispositif de prélèvement 3 sur le corps 1 dans une position de stockage dans laquelle l'étanchéité à l'air du réservoir 2 est renforcée, ledit dispositif de prélèvement étant déplacé ultérieurement dans une position de distribution. En effet, en position de distribution, l'étanchéité statique à l'air du réservoir 2 présentant une dépression peut être insuffisante, notamment en présence d'un trou d'évent 26, pour garantir le maintien de cette dépression à l'issue d'un stockage prolongé.

En outre, toujours pour améliorer le maintien de la dépression dans le temps, la soupape 15 de remplissage peut être recouverte de façon réversible par un opercule d'étanchéité 28. En relation avec la figure 5a, l'opercule 28 est soudé dans un embrèvement pratiqué sur l'extrémité libre de l'enjoliveur 20 de sorte que ledit opercule recouvre totalement la soupape 15, ledit opercule présentant un bord libre 28a permettant son retrait en vue du remplissage initial.

En relation avec les figures 3 et 4, la pompe 3 est monté coulissante par rapport au corps 1 entre une position supérieure de stockage dans laquelle le trou d'évent 26 est occulté (figure 4) et une position inférieure de distribution (figure 3) dans laquelle ledit trou d'évent est libre afin de permettre la reprise d'air.

En particulier, le corps 3 est monté dans le manchon 27 avec un serrage adapté pour permettre le coulissement, le passage entre ces positions étant réalisé par appui sur le bouton poussoir 4 lors du premier actionnement de la pompe 3. Le manchon 27 présente une marche 27b délimitant un diamètre supérieur 27c de montage étanche du corps 5 afin d'occulter le trou d'évent 26 et un diamètre inférieur augmenté 27a laissant libre ledit trou d'évent, ledit corps de pompe présentant une butée 29 de fin de course de coulissement dans le manchon 27.

## Revendications

1. Procédé de distribution d'un produit fluide au moyen d'un flacon de distribution comprenant un corps rigide (1) dans lequel un réservoir (2) de conditionnement dudit produit est formé, ledit flacon comprenant en outre un dispositif de prélèvement (3) du produit conditionné qui est monté de façon étanche sur ledit corps, ledit procédé prévoyant :
- préalablement au remplissage initial du réservoir (2) en produit, de mettre ledit réservoir vide en communication avec un dispositif d'aspiration d'air et d'activer ledit dispositif afin de créer une dépression à l'intérieur dudit réservoir ;
- de réaliser ultérieurement le remplissage initial du réservoir (2) par mise en communication étanche d'une source de produit (16) avec ie réservoir (2) de sorte que la dépression induise le remplissage dudit réservoir par aspiration du produit contenu dans ladite source ;
- de distribuer le produit conditionné par actionnement du dispositif de prélèvement (3).
ledit procédé étant **caractérisé en ce que** le corps (1) est équipé d'une soupape (15) de remplissage dudit réservoir qui est agencée pour permettre la mise en communication de la source de produit (16) avec ledit réservoir, le remplissage initial du réservoir (2) en produit étant réalisé par mise en communication étanche de la source (16) de produit avec ledit réservoir par l'intermédiaire de la soupape (15).

2. Procédé de distribution selon la revendication 1, **caractérisé en ce que** le dispositif d'aspiration d'air comprend une cloche à vide dans laquelle le flacon de distribution est disposé, le montage étanche du dispositif de prélèvement (3) sur le corps (1) étant réalisé après activation de ladite cloche.

3. Procédé de distribution selon la revendication 1, **caractérisé en ce que** le dispositif d'aspiration d'air est mis en communication avec le dispositif de prélèvement (3) après son montage étanche sur le corps (1), l'aspiration de l'air du réservoir (2) étant réalisée au travers dudit dispositif de prélèvement.

4. Procédé de distribution selon la revendication 1, **caractérisé en ce que** le dispositif d'aspiration d'air est mis en communication avec la soupape (15) après montage étanche du dispositif de prélèvement (3) sur le corps (1), l'aspiration de l'air du réservoir (2) étant réalisée au travers de ladite soupape.

5. Procédé de distribution selon l'une quelconque des revendications 1 à 4, **caractérisé en ce que** la source de produit (16) comprend un réservoir source sur lequel est disposé un tube de prélèvement (17), le remplissage du réservoir (2) de conditionnement étant réalisé par montage dudit tube en appui étanche sur la soupape (15) qui est agencée pour s'ouvrir de façon réversible afin d'aspirer du produit depuis ledit réservoir source dans ledit réservoir de conditionnement.

6. Procédé de distribution selon la revendication 5, **caractérisé en ce que** le réservoir source est formé à l'intérieur d'une poche souple.

7. Procédé de distribution selon l'une quelconque des revendications 1 à 6, **caractérisé en ce qu'**il prévoit, préalablement au remplissage initial du réservoir (2) en produit, le montage du dispositif de prélèvement (3) sur le corps (1) dans une position de stockage dans laquelle l'étanchéité à l'air dudit réservoir est renforcée, ledit dispositif de prélèvement étant déplacé ultérieurement dans une position de distribution.

8. Procédé de distribution selon la revendication 7, **caractérisé en ce que** le dispositif de prélèvement (3) est monté coulissant par rapport au corps (1) entre une position supérieure de stockage et une position inférieure de distribution, le passage entre ces positions étant réalisé lors du premier actionnement du dispositif de prélèvement (3).

9. Procédé de distribution selon la revendication 7 ou 8, **caractérisé en ce que** le dispositif de prélèvement (3) comprend un trou d'évent (26) qui est agencé pour permettre de compenser le volume de produit prélevé dans le réservoir (2) par de l'air, ledit trou d'évent étant occulté en position de stockage et libre en position de distribution afin de permettre la reprise d'air.

10. Procédé de distribution selon l'une quelconque des revendications 1 à 7, **caractérisé en ce que** le produit est prélevé sans reprise d'air dans le réservoir (2) de conditionnement de sorte à créer une dépression dans ledit réservoir qui augmente au fur et à mesure de la distribution, ledit procédé prévoyant en outre le remplissage ultérieur dudit réservoir par mise en communication étanche de la source de produit (16) avec ledit réservoir par l'intermédiaire de la soupape (15) de sorte que la dépression induise le remplissage dudit réservoir par aspiration du produit contenu dans ladite source.

11. Procédé de distribution selon l'une quelconque des revendications 1 à 9, **caractérisé en ce que** le produit est prélevé avec reprise d'air dans le réservoir (2) de conditionnement de sorte à empêcher le remplissage ultérieur dudit réservoir par aspiration.

12. Procédé de distribution selon l'une quelconque des revendications 1 à 11, **caractérisé en ce qu'**il prévoit l'association d'une étiquette sur le corps (1) au moment du remplissage initial.

13. Flacon de distribution d'un produit fluide comprenant un corps rigide (1) dans lequel un réservoir (2) destiné au conditionnement dudit produit est formé, ledit flacon comprenant en outre un dispositif de prélèvement (3) dudit produit conditionné qui est monté de façon étanche sur ledit corps, le réservoir (2) étant vide de produit et présentant une dépression d'air qui est agencée pour pouvoir réaliser ultérieurement le remplissage initial du réservoir (2) en produit par mise en communication étanche d'une source de produit (16) avec ledit réservoir de sorte que ladite dépression induise le remplissage dudit réservoir par aspiration du produit contenu dans ladite source, ledit flacon étant **caractérisé en ce que** le corps (1) est équipé d'une soupape (15) de remplissage dudit réservoir qui est corps (1) est équipé d'une soupape (15) de remplissage dudit réservoir qui est agencée pour permettre la mise en communication de la source de produit (16) avec ledit réservoir.

14. Flacon de distribution selon la revendication 13, **caractérisé en ce que** le dispositif de prélèvement (3) est du type sans reprise d'air dans le réservoir (2) de conditionnement en compensation du volume de produit distribué.

15. Flacon de distribution selon la revendication 13, **caractérisé en ce que** le dispositif de prélèvement (3) comprend un trou d'évent (26) qui est agencé pour permettre de compenser le volume de produit prélevé dans le réservoir (2) par de l'air.

16. Flacon de distribution selon la revendication 15, **caractérisé en ce que** le dispositif de prélèvement (3) est monté sur le corps (1) dans une position de stockage dans laquelle le trou d'évent (26) est occulté, ledit dispositif de prélèvement étant déplaçable par rapport au corps (1) dans une position de distribution dans laquelle ledit trou d'évent est libre afin de permettre la reprise d'air.

17. Flacon de distribution selon l'une quelconque des revendications 13 à 16, **caractérisé en ce que** la soupape (15) de remplissage est recouverte de façon réversible par un opercule d'étanchéité (28).

18. Flacon de distribution selon l'une quelconque des revendications 13 à 17, **caractérisé en ce que** la soupape (15) présente un siège (19) qui est mobile et/ou déformable entre un état stable de fermeture étanche du réservoir (2) de conditionnement et un état contraint de mise en communication étanche de la source de produit (16) avec ledit réservoir.

19. Flacon de distribution selon l'une quelconque des revendications 13 à 18, **caractérisé en ce que** la soupape (15) de remplissage est disposée sur l'extrémité inférieure du corps (1).

## Patentansprüche

1. Verfahren zur Abgabe eines flüssigen Produkts mittels einer Abgabeflasche, umfassend einen starren Körper (1), in dem ein Behälter (2) zum Aufbewahren des Produkts gebildet ist, wobei die Flasche ferner eine Entnahmevorrichtung (3) des aufbewahrten Produkts umfasst, die dicht auf dem Körper befestigt ist, wobei das Verfahren vorsieht:
- vor dem erstmaligen Befüllen des Behälters (2) mit dem Produkt, Leeren des Behälters in Verbindung mit einer Luftansaugvorrichtung und Aktivieren der Vorrichtung zur Herstellung eines Unterdrucks im Inneren des Behälters;
- anschließend Durchführen des erstmaligen Befüllens des Behälters (2) durch dichtes Verbinden einer Quelle des Produkts (16) mit dem Behälter (2), sodass der Unterdruck das Befüllen des Behälters durch Ansaugen des in der Quelle enthaltenen Produkts bewirkt;
- Abgeben des aufbewahrten Produkts durch Betätigung der Entnahmevorrichtung (3);
wobei das Verfahren **dadurch gekennzeichnet ist, dass** der Körper (1) ausgestattet ist mit einem Ventil (15) zum Befüllen des Behälters, das so beschaffen ist, dass es das Verbinden der Quelle des Produkts (16) mit dem Behälter ermöglicht, wobei das erstmalige Befüllen des Behälters (2) mit dem Produkt durch das dichte Verbinden der Quelle (16) des Produkts mit dem Behälter über das Ventil (15) ausgeführt wird.

2. Abgabeverfahren nach Anspruch 1, **dadurch gekennzeichnet, dass** die Luftansaugvorrichtung eine Vakuumglocke umfasst, in der die Abgabeflasche angeordnet ist, wobei das dichte Befestigen der Entnahmevorrichtung (3) auf dem Körper (1) nach Aktivierung der Glocke ausgeführt wird.

3. Abgabeverfahren nach Anspruch 1, **dadurch gekennzeichnet, dass** die Luftansaugvorrichtung mit der Entnahmevorrichtung (3) nach deren dichter Befestigung auf dem Körper (1) verbunden wird, wobei das Ansaugen der Luft des Behälters (2) durch die Entnahmevorrichtung hindurch durchgeführt wird.

4. Abgabeverfahren nach Anspruch 1, **dadurch gekennzeichnet, dass** die Luftansaugvorrichtung mit dem Ventil (15) nach dichter Befestigung der Entnahmevorrichtung (3) auf dem Körper (1) verbunden wird, wobei das Ansaugen der Luft des Behälters (2) durch das Ventil hindurch durchgeführt wird.

5. Abgabeverfahren nach einem der Ansprüche 1 bis 4, **dadurch gekennzeichnet, dass** die Quelle des Produkts (16) einen Quellen-Behälter umfasst, auf dem ein Entnahmerohr (17) angeordnet ist, wobei das Befüllen des Behälters (2) zum Aufbewahren durch dichtes Anbringen dieses Rohrs auf dem Ventil (15) durchgeführt wird, das so beschaffen ist, dass es sich reversibel öffnet, sodass das Produkt von dem Quellen-Behälter in den Aufbewahrungsbehälter angesaugt wird.

6. Abgabeverfahren nach Anspruch 5, **dadurch gekennzeichnet, dass** der Quellen-Behälter im Inneren eines flexiblen Beutels gebildet wird.

7. Abgabeverfahren nach einem der Ansprüche 1 bis 6, **dadurch gekennzeichnet, dass** es vorsieht, vor dem erstmaligen Befüllen des Behälters (2) mit dem Produkt die Entnahmevorrichtung (3) auf dem Körper (1) in einer Speicherposition zu befestigen, in der die Luftdichtigkeit des Behälters verstärkt ist, und die Entnahmevorrichtung danach in eine Abgabeposition zu verschieben.

8. Abgabeverfahren nach Anspruch 7, **dadurch gekennzeichnet, dass** die Entnahmevorrichtung (3) verschiebbar gegenüber dem Körper (1) zwischen einer oberen Speicherposition und einer unteren Abgabeposition befestigt ist, wobei der Übergang zwischen diesen beiden Positionen bei der ersten Betätigung der Entnahmevorrichtung (3) ausgeführt wird.

9. Abgabeverfahren nach Anspruch 7 oder 8, **dadurch gekennzeichnet, dass** die Entnahmevorrichtung (3) ein Luftloch (26) umfasst, das so beschaffen ist, dass es ermöglicht, das Volumen von entnommenem Produkt in dem Behälter (2) durch Luft auszugleichen, wobei das Luftloch in der Speicherposition bedeckt ist und in der Abgabeposition frei ist, sodass die Luftaufnahme ermöglicht wird.

10. Abgabeverfahren nach einem der Ansprüche 1 bis 7, **dadurch gekennzeichnet, dass** das Produkt entnommen wird, ohne dass in dem Behälter (2) zum Aufbewahren Luft aufgenommen wird, sodass ein Unterdruck in dem Behälter geschaffen wird, der im Zuge der Abgabe zunimmt, wobei das Verfahren ferner das anschließende Befüllen des Behälters durch dichtes Verbinden der Quelle des Produkts (16) mit dem Behälter über das Ventil (15) vorsieht, sodass der Unterdruck das Befüllen des Behälters durch Ansaugen des in der Quelle enthaltenen Produkts bewirkt.

11. Abgabeverfahren nach einem der Ansprüche 1 bis 9, **dadurch gekennzeichnet, dass** das Produkt unter Luftaufnahme in den Behälter (2) zum Aufbewahren entnommen wird, sodass das anschließende Befüllen des Behälters durch Ansaugen verhindert wird.

12. Abgabeverfahren nach einem der Ansprüche 1 bis 11, **dadurch gekennzeichnet, dass** es das Anbringen eines Etiketts auf dem Körper (1) zum Zeitpunkt des erstmaligen Befüllens vorsieht.

13. Abgabeflasche eines flüssigen Produkts, umfassend einen starren Körper (1), in dem ein Behälter (2) zum Aufbewahren dieses Produkts gebildet ist, wobei die Flasche ferner eine Entnahmevorrichtung (3) des aufbewahrten Produkts umfasst, die dicht auf dem Körper befestigt ist, wobei der Behälter (2) kein Produkt enthält und einen Luftunterdruck aufweist, der so beschaffen ist, dass später das erstmalige Befüllen des Behälters (2) mit dem Produkt durchgeführt werden kann, indem eine Quelle des Produkts (16) mit dem Behälter dicht verbunden wird, sodass dieser Unterdruck das Befüllen des Behälters durch Ansaugen des in der Quelle enthaltenen Produkts bewirkt, wobei die Flasche **dadurch gekennzeichnet ist, dass** der Körper (1) ausgestattet ist mit einem Ventil (15) zum Befüllen des Behälters, das so beschaffen ist, dass es das Verbinden der Quelle des Produkts (16) mit dem Behälter ermöglicht.

14. Abgabeflasche nach Anspruch 13, **dadurch gekennzeichnet, dass** die Entnahmevorrichtung (3) keine Luftaufnahme in den Behälter (2) zum Aufbewahren als Ausgleich des Volumens des abgegebenen Produkts vorsieht.

15. Abgabeflasche nach Anspruch 13, **dadurch gekennzeichnet, dass** die Entnahmevorrichtung (3) ein Luftloch (26) umfasst, das so beschaffen ist, dass es ermöglicht, das Volumen von entnommenem Produkt in dem Behälter (2) durch Luft auszugleichen.

16. Abgabeflasche nach Anspruch 15, **dadurch gekennzeichnet, dass** die Entnahmevorrichtung (3) auf dem Körper (1) in einer Speicherposition befestigt ist, in der das Luftloch (26) bedeckt ist, wobei die Entnahmevorrichtung gegenüber dem Körper (1) in eine Abgabeposition verschiebbar ist, in der das Luftloch frei ist, sodass die Luftaufnahme ermöglicht wird.

17. Abgabeflasche nach einem der Ansprüche 13 bis 16, **dadurch gekennzeichnet, dass** das Ventil (15) zum Befüllen reversibel mit einer Dichtungsabdeckung (28) versehen ist.

18. Abgabeflasche nach einem der Ansprüche 13 bis 17, **dadurch gekennzeichnet, dass** das Ventil (15} einen Sitz (19) aufweist, der beweglich und/oder verformbar zwischen einem stabilen Zustand des dichten Verschließens des Behälters (2) zum Aufbewahren und einem gespannten Zustand des dichten Verbindens der Quelle des Produkts (16) mit dem Behälter ist.

19. Abgabeflasche nach einem der Ansprüche 13 bis 18, **dadurch gekennzeichnet, dass** das Ventil (15) zum Befüllen auf dem unteren Ende des Körpers (1) befestigt ist.

## Claims

1. Method for dispensing a fluid product by means of a dispensing bottle comprising a rigid body (1) into which a reservoir (2) for packaging said product is formed, said bottle additionally comprising a device (3) for collecting said packaged product mounted in a sealed manner on said body, said method including:
- prior to the initial filling of reservoir (2) with product, placing said empty reservoir in communication with an air suction device and actuating said device in order to create a negative pressure inside said reservoir;
- subsequently performing the initial filling of the reservoir (2) by placing a product source (16) in sealed communication with the reservoir (2) such that the negative pressure causes filling of said reservoir by suction of the product contained in said source;
- dispensing the packaged product by actuating the collecting device (3),
said process being **characterised in that** the body (1) is equipped with a valve (15) for filling said reservoir which is arranged so as to enable the product source (16) to be placed in communication with said reservoir, the initial filling of the reservoir (2) with product being performed by placing the product source (16) in sealed communication with said reservoir by means of the valve (15).

2. Dispensing method according to claim 1, **characterised in that** the air suction device comprises a vacuum chamber into which the dispensing bottle is placed, the sealed assembly of the collecting device (3) on the body (1) being performed after activation of said chamber.

3. Dispensing method according to claim 1, **characterised in that** the air suction device is placed in communication with the collecting device (3) after sealed assembly thereof on the body (1), the suction of air from reservoir (2) being carried out through said collecting device.

4. Dispensing method according to claim 1, **characterised in that** the air suction device is placed in communication with the valve (15) after sealed assembly of the collecting device (3) on the body (1), the suction of air from reservoir (2) being carried out through said valve.

5. Dispensing method according to any one of claims 1 to 4, **characterised in that** the product source (16) comprises a source reservoir onto which a collecting tube (17) is placed, the filling of packaging reservoir (2) being carried out by assembling said tube in sealed contact on the valve (15), which is arranged so as to reversibly open in order to suck product from said source reservoir into said packaging reservoir.

6. Dispensing method according to claim 5, **characterised in that** the source reservoir is formed inside a flexible pouch.

7. Dispensing method according to any one of claims 1 to 6, **characterised in that** it includes, prior to the initial filling of the reservoir (2) with product, the assembling of the collecting device (3) on the body (1) in a storage position in which the airtightness of said reservoir is strengthened, said collecting device being later moved into a dispensing position.

8. Dispensing method according to claim 7, **characterised in that** the collecting device (3) is slidingly mounted relative to the body (1) between an upper storage position and a lower dispensing position, the passage between these positions being carried out during the first actuation of the collecting device (3).

9. Dispensing method according to claim 7 or 8, **characterised in that** the collecting device (3) comprises a vent hole (26) that is arranged to allow compensation for the volume of product collected into the reservoir (2) by air, said vent hole being concealed in the storage position and free in the dispensing position in order to allow the intake of air.

10. Dispensing method according to any one of claims 1 to 7, **characterised in that** the product is collected without the intake of air into the packaging reservoir (2) such that a negative pressure is created in said reservoir which increases as dispensing progresses, said method further including the subsequent filling of said reservoir by placing the product source (16) into sealed communication with said reservoir by means of the valve (15) such that the negative pressure causes filling of said reservoir by suction of the product contained in said source.

11. Dispensing method according to any one of claims 1 to 9, **characterised in that** the product is collected with an intake of air into the packaging reservoir (2) such as to prevent subsequent filling of said reservoir by suction.

12. Dispensing method according to any one of claims 1 to 11, **characterised in that** it includes the association of a label on the body (1) at the time of initial filling.

13. A bottle for dispensing a fluid product comprising a rigid body (1) into which a reservoir (2) intended for the packaging of said product is formed, said bottle additionally comprising a device (3) for collecting said packaged product mounted in sealed manner on said body, reservoir (2) being empty of product and having a negative pressure, which is arranged so as to be able to subsequently carry out the initial filling of the reservoir (2) with product by placing a product source (16) in sealed communication with said reservoir such that the negative pressure causes filling of said reservoir by suction of product contained in said source, said bottle being **characterised in that** the body (1) is equipped with a valve (15) for filling said reservoir, which is arranged so as to enable the product source (16) to be placed in communication with said reservoir.

14. Dispensing bottle according to claim 13, **characterised in that** the collecting device (3) is of the type without air intake into the packaging reservoir (2) in compensation for the volume of product dispensed.

15. Dispensing bottle according to claim 13, **characterised in that** the collecting device (3) comprises a vent hole (26) that is arranged to allow compensation for the volume of product collected into the reservoir (2) by air.

16. Dispensing bottle according to claim 15, **characterised in that** the collecting device (3) is mounted on the body (1) in a storage position in which the vent hole (26) is concealed, said collecting device being movable relative to the body (1) into a dispensing position in which said vent hole is free in order to enable intake of air.

17. Dispensing bottle according to any one of claims 13 to 16, **characterised in that** the filling valve (15) is reversibly covered by a sealing cap (28).

18. Dispensing bottle according to any one of claims 13 to 17, **characterised in that** the valve (15) has a seat (19) that is mobile and/or deformable between a stable state of sealed closure of the packaging reservoir (2) and a constrained state of placing the product source (16) in sealed communication with said reservoir.

19. Dispensing bottle according to any one of claims 13 to 18, **characterised in that** the filling valve (15) is arranged on the lower end of the body (1).
